(19) Europäisches Patentamt
European Patent Office
Office européen des brevets

(11) Publication number: **0 004 409**
**B1**

# (12) EUROPEAN PATENT SPECIFICATION

(45) Date of publication of patent specification: **21.04.82**

(21) Application number: **79200141.4**

(22) Date of filing: **22.03.79**

(51) Int. Cl.³: **C 07 C 11/12**, C 07 C 2/38, B 01 J 31/28 //B01J31/24

## (54) A process for preparing 1,7-octadiene.

(30) Priority: **27.03.78 US 890117**

(43) Date of publication of application:
**03.10.79 Bulletin 79/20**

(45) Publication of the grant of the patent:
**21.04.82 Bulletin 82/16**

(84) Designated Contracting States:
**DE FR GB NL**

(56) References cited:
**GB - A - 1 341 324**
**US - A - 3 732 328**
**US - A - 3 823 199**

**JOURNAL OF ORGANOMETALLIC CHEMISTRY vol. 55, July 16, 1973, LAUSANNE (CH) P. ROFFIA et al.: "Catalysis by Palladium Salts IV. Selective hydrogenation with formic acid in the palladium catalysed dimerisation of 1,3-butadiene; Synthesis of 1,7-octadiene", pages 405—407.**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Nozaki, Kenzie**
**1407 Lakecliff**
**Houston Texas 77077 (US)**

(74) Representative: **Puister, Antonius Tonnis, Mr. et al,**
**P.O. Box 302**
**NL-2501 CH The Hague (NL)**

**EP 0 004 409 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## A process for preparing 1,7-octadiene

The invention relates to a process for preparing 1,7-octadiene by hydrodimerizing 1,3-butadiene in the presence of a catalytic amount of palladium or a palladium compound, a tertiary phosphine, formic acid, a base which can neutralize formic acid and optionally a solvent.

Linear dimerization of 1,3-butadiene provides a source of $C_8$ unsaturated hydrocarbon intermediates useful for the synthesis of diacids, diesters, diols or diamines. A particularly preferred dimer is 1,7-octadiene which has terminal double bonds and allows the production of products having only terminal functional groups.

U.S. Patent Specification No. 3,732,328 discloses the preparation of mixtures of octadienes, primarily 1,6-octadiene, by reacting 1,3-butadiene in the presence of a palladium compound, a polar solvent, a reducing agent and a tertiary phosphine. Trialkyl-, mixed alkylaryl- or triaryl-phosphines are mentioned as useful, specifically noting triphenylphosphine.

U.S. Patent Specification No. 3,823,199 also discloses the preparation of mixtures of octadienes, primarily 1,6-octadiene, by reacting 1,3-butadiene in the presence of palladium metal or a palladium compound, a non-polar solvent, a reducing agent and a tertiary phosphine. Tricycloalkylphosphines are stated as being useful but stated to be equivalent to trialkylphosphines. Specifically mentioned are triethyl- and tributylphosphine and trialkyl-phosphines in which the alkyl group contains other substituents, e.g. a phenyl group as in tribenzylphosphine.

British Patent Specification No. 1,341,324 discloses the production of 1,6- and/or 1,7-octadienes by contacting a conjugated di-olefine with metallic palladium or with a palladium compound in an amine solvent in the presence of a reducing agent.

Journal of Organometallic Chemistry, 55 (1973) 405—407 discloses the use of a triphenylphosphine-zero valent palladium complex catalyst in benzene in the presence of formic acid to dimerize butadiene. Although a 75% butadiene conversion is reported, only 22% of the product was 1,7-octadiene. No phosphine other than the triphenylphosphine was mentioned.

Tetrahydron Letters No. 2 (1972) pp. 163—164 discloses a process as described in the beginning, namely the production of mixtures of octadienes primarily by reacting butadiene in the presence of palladium salts, an organic base, formic acid and a phosphine. No particular phosphine was noted as selectively producing 1,7-octadiene.

It has now surprisingly been found that extremely high selectivities to 1,7-octadiene are obtained if in the hydrodimerization of 1,3-butadiene as described in the beginning the tertiary phosphine used has a special formula.

Accordingly, the invention provides a process for preparing 1,7-octadiene by hydrodimerizing 1,3-butadiene in the presence of a catalytic amount of palladium or a palladium compound, a tertiary phosphine, formic acid, a base which can neutralize formic acid and optionally a solvent, characterized in that the tertiary phosphine has the formula:

$$R_1R_2R_3P$$

wherein $R_1$ is a branched alkyl group having from 3 to 10 carbon atoms with branching occurring at a carbon atom no more than two carbon atoms from the phosphorus atom and $R_2$ and $R_3$ are $R_1$ or independently are alkyl, alkenyl or aryl groups having from 1 to 10 carbon atoms.

Solvents are not essential to the process of this invention, but a good organic solvent can promote the rate of reaction by a factor of two or more.

The non-polar solvents (disclosed in U.S. Patent Specification No. 3,823,199) such as paraffinic, cycloparaffinic or aromatic are useful in the process of this invention. The solvent can be a paraffin or cycloparaffin containing 5 to 16 carbon atoms per molecule, such as hexane, dodecane, pentadecane, cyclohexane, methylcyclohexane and the like. Suitable solvents also include aromatic hydrocarbons such as benzene, lower alkyl substituted aromatic hydrocarbons such as toluene, m-, p- and o-xylene, halogenated aromatic hydrocarbons including chloro, bromo and iodo substituted, such as chlorobenzene and the like. Halogenated lower aliphatic compounds such as chloroform, methylene chloride, carbon tetrachloride and the like may be used; in particular chloroform is preferred.

Further useful solvents are the amine solvents disclosed in British Patent Specification No. 1,341,324. A wide range of amines are useful provided that they are liquid under reaction conditions. Tertiary amines are preferred to primary and secondary amines. Suitable amine solvents include alkylamines, cycloalkylamines, arylamines and heterocyclic amines such as morpholine, pyridine, piperazine and piperidine. Examples of these classes of amines are the lower alkylamines containing 2 to 6 carbon atoms in each alkyl group such as triethylamine; monocyclohexylamine, and N-alkylcyclohexylamines containing up to 12 carbon atoms per molecule; aniline and N-alkylanilines containing up to 12 carbon atoms per molecule and N-alkylmorpholines containing up to 12 carbon atoms per molecule. The preferred amine solvent is pyridine.

Further useful solvents are those of moderate co-ordinating ability, and which include nitriles such as lower alkanenitriles and hydrocarbon aromatic nitriles including acetonitrile, benzonitrile and the like,

amides including benzamide, acetamide, mono- and di-substituted amides where the substituent is preferably lower alkyl. Suitable substituted amides include N-methylacetamide, N,N-dimethylacetamide and dimethylformamide. Dialkyl sulfoxides such as dimethyl sulfoxide and sulfones such as sulfolane and alkyl-substituted sulfolane are satisfactory. Simple ethers such as the di(lower alkyl)ethers including dimethyl ether, diethyl ether, and the like function satisfactorily. Hydrocarbon aromatic ethers such as the lower alkyl phenyl ethers may also be used, and include methyl phenyl ether (anisole), ethyl phenyl ether (phenetole) and the like. Cyclic, saturated hydrocarbon ethers such as tetrahydrofuran, tetrahydropyran and the like are also suitable solvents. Lower alkyl diethers such as dimethoxyethane, and the like may be used. In addition, the cyclic diethers such as 1,4-dioxane are also suitable as solvents.

Simple lower alkyl esters of lower alkanoic acids such as ethyl acetate, methyl acetate, methyl butyrate, ethylene carbonate and propylene carbonate and the like are also suitable solvents of moderate co-ordinating ability. Ketones, including lower aliphatic ketones such as methyl ether ketone and hydrocarbon aromatic ketones such as acetophenone are also satisfactory solvents. Lower alkanols and alkanediols such as 2-propanol, ethylene glycol and the like may be used if desired. The preferred solvents of moderate co-ordinating ability include nitriles, formamides, such as dimethylformamide, di(lower alkyl)ethers, lower alkyl phenyl ethers, simple lower alkyl esters of lower alkanoic acids, ketones and lower alkanols.

Particularly useful solvents utilized in this invention include pyridine, benzene, dimethylformamide, chlorobenzene, anisole, N,N-dimethylacetamide, ethyl acetate, 2-propanol, benzonitrile, chloroform, methyl ethyl ketone, acetonitrile, diethyl ether, acetophenone, toluene, ethylene glycol, ethylene carbonate, and sulfolane.

The preferred organic solvents will have from 1 to 20 carbon atoms per molecule. Particularly desired solvents are those which give two-phase systems which allow easy product separation such as, for example, nitromethane, ethylene carbonate and propylene carbonate.

The amount of solvent added should be sufficient to dissolve the catalyst.

The formic acid is utilized as a source of hydrogen for the process. It is present in the reaction mixture as a salt of the base promoter utilized. It is thought that dissociation of the formic acid-base salt provides a suitable amount of formic acid necessary to provide the required hydrogen. Excess free acid present in the reaction mixture has an inhibitory effect on the reaction.

It is desirable that some formic acid, as the salt, be present during the entire course of the reaction. When operating the process batchwise, this can be accomplished by adding a stoichiometric amount of formic acid initially, 1 mol of formic acid for every 2 mol of 1,3-butadiene, or by continuously or periodically adding additional amounts of formic acid. It is highly desirable, however, that the ratio of base to formic acid present in the reaction medium never be less than 1.

The base must be one which can neutralize formic acid according to the reaction:

$$HCOOH+B \rightarrow HCOO^{-}HB^{+}.$$

The base may be either insoluble or soluble in the reaction medium.

The base may be organic or inorganic. Suitable organic bases typically have dissociation constants greater than $10^{-8}$ and include tertiary amines such as triethylamine, tributylamine, dimethylethylamine, lutidine, tripropylamine, N-methylmorpholine, N-methyl-2,2,6,6-tetramethylpiperidine, 2,8-(dimethylamino)naphthalene and the like. Small amounts of water may be present.

Suitable inorganic bases include ammonia, the hydroxide bases such as sodium hydroxide, potassium hydroxide, calcium hydroxide; ammonium hydroxide; the carbonates and bicarbonates such as sodium carbonate, sodium hydrogen carbonate, potassium carbonate, potassium hydrogen carbonate, calcium carbonate and the like; the weak bases such as sodium acetate, potassium acetate, ammonium carbonate, ammonium acetate and the like. When the inorganic bases are utilized, small amounts of water may be present.

The molar ratio of base to formic acid should be at least equal to 1. When organic bases are utilized, excess base may be utilized as a solvent or the amine-base salt may be used as the solvent.

The catalyst used in the process of this invention is palladium complexed with the tertiary phosphine ligand in question or a palladium compound complexed with the tertiary phosphine ligand in question. The palladium may be in any of its possible valence states, e.g. 0, +2, etc. Suitable palladium compounds include the palladium carboxylates, particularly palladium carboxylates derived from alkanoic acids containing up to six carbon atoms per molecule such as palladium acetate, complexes such as palladium acetylacetonate, bis-benzonitrile palladium (II) and lithium palladous chloride as well as the palladium halides, nitrates and sulphates such as palladous chloride and palladium nitrate $(Pd(NO_3)_2(OH_2)$ and palladium sulphate. Other suitable palladium compounds include palladium-phosphine complexes, such as $Pd(R_3P)_2$ or $Pd(R_3P)_3$. The palladium is present in the reaction mixture in catalytic amounts; preferably from 1 to $10^{-6}$ molar and more preferably from $10^{-1}$ to $10^{-4}$ molar.

By "branched" is meant that the $\alpha$- and $\beta$-carbon atoms, relative to the phosphorus atom, of the alkyl group in the tertiary phosphine of the formula $R_1R_2R_3P$ may not both be —$CH_2$— linkages.

Illustrative of the $R_1$ moiety are isopropyl, sec-butyl, tert-butyl, isobutyl, neopentyl, 1-methylbutyl, tert-pentyl, 2-methylbutyl, 1-methylpentyl, 1,1-dimethylbutyl and 2,2-dimethylpropyl.

Illustrative of $R_2$ and $R_3$ are, for example, methyl, ethyl, propyl, butyl, pentyl, hexyl, octyl, nonyl and decyl groups, allyl, crotyl and methallyl groups and phenyl, tolyl, xylyl, ethylphenyl and propylphenyl groups. Two or more of the instant phosphines may be used in the same reaction or another phosphine may be replaced by reacting in situ one of the instant phosphines. The molar ratio of tertiary phosphine to palladium is preferably at least 1. Preferably the molar ratio of phosphine to palladium ranges from 1:1 to 20:1 and preferably from 2:1 to 5:1. The use of the phosphines according to the invention provides extremely high selectivities to 1,7-octadiene.

The addition of carbon dioxide to the reaction system has been found to increase the extent of butadiene conversion, but does not affect the selectivity. When it is desired to use carbon dioxide to increase the conversion rate, the partial pressure of the $CO_2$ in the reaction system may be from about 0.07 to about 0.7 MPa (about 10 to about 100 psia). Since carbon dioxide is a by-product of the process, it is possible to generate sufficient carbon dioxide *in situ* to enhance the conversion rates.

The process can be either continuous or batch. The reaction temperature of the process is not critical, however, it is preferred to maintain the reaction between about 0 to about 100°C, preferably between about 20 to about 70°C. The process is conducted under a sufficient pressure to maintain liquid phase conditions at the reaction temperature. Typically the pressure is autogenous. The process of this invention is particularly useful when applied to a 1,3-butadiene/isobutene/n-butenes (BBB) stream from an oil pyrolysis unit. These BBB streams are the $C_4$ cut from a thermal cracking unit typically containing 30—40%, 1,3-butadiene, 20—35% isobutene and 20—30% n-butenes and many other minor components.

The following Examples further illustrate the invention.

Examples 1—12 and comparative experiments A—D

To an 80 ml glass-lined autoclave were charge palladium ($2.7 \times 10^{-5}$ mol) as a 10% water solution of $Pd(NO_3)_2(OH)_2$, the appropriate phosphine ($5.4 \times 10^{-5}$ mol) listed in Table I, triethylamine-formic acid salt (2.5 g) pyridine (10 ml) and 1,3-butadiene (2 g). The stirred reactor was heated to 40°C for 2 hours, cooled and the product was analyzed by gas chromatography. The results are shown in Table I. The figures and the capitals in the left hand column refer to the Examples and the Comparative Experiments, respectively.

Table I

| Example or Comparative Experiment | Phosphine | Butadiene Conversion, % | Selectivity to 1,7-octadiene, % |
|---|---|---|---|
| 1 | $(iso\text{-}propyl)_3P$ | 30.0 | 98 |
| A | $(n\text{-}propyl)_3P$ | 24.0 | 92 |
| 2 | $(sec\text{-}butyl)_3P$ | 25.0 | 98 |
| 3 | $(iso\text{-}butyl)_3P$ | 8.0 | 94 |
| 4 | $(tert\text{-}butyl)_3P$ | 10.0 | 95 |
| 5 | $(sec\text{-}butyl)_2phenylP$ | 15.0 | 94 |
| 6 | $(tert\text{-}butyl)_2phenylP$ | 45.0 | 91 |
| 7 | $(tert\text{-}butyl)_2\text{-}n\text{-}butylP$ | 15.0 | 98 |
| 8 | $(tert\text{-}butyl)_2allylP$ | 70.0 | 98 |
| 9 | $tert\text{-}butyl(iso\text{-}propyl)_2P$ | 19.0 | 97 |
| 10 | $tert\text{-}butyl(n\text{-}propyl)_2P$ | 10.0 | 95 |
| 11 | $(tert\text{-}butyl)_2methylP$ | 26.7 | 92 |
| 12 | tert-butyldimethylP | 24.0 | 92 |
| B | $(n\text{-}butyl)_3P$ | 21.0 | 89 |
| C | $(ethyl)_2phenylP$ | 15.0 | 87 |
| D | $(benzyl)_3P$ | 15.0 | 93 |

Examples 13 and 14 and comparative experiments E to H

To an 80 ml glass-lined autoclave were charged palladium ($2.7 \times 10^{-5}$ mol) as palladium acetate, the appropriate phosphine ($5.4 \times 10^{-5}$ mol) listed in Table II, triethylamineformic acid salt (2.5 g), 1,3-butadiene (2 g) and pyridine (10 ml). The stirred reactor was heated to 60°C for 1 hour. The reactor was then cooled and the product was analyzed by gas chromatography. The results are shown in Table II. The figures and the capitals in the left hand column refer to the Examples and the Comparative Experiments, respectively.

Table II

| Example or Comparative Experiment | Phosphine | Butadiene Conversion, % | Selectivity to 1,7-octadiene, % |
|---|---|---|---|
| 13 | $(iso\text{-}propyl)_3P$ | 95.0 | 98 |
| E | $(n\text{-}propyl)_3P$ | 95.0 | 90 |
| 14 | $(iso\text{-}butyl)_3P$ | 30.0 | 94 |
| F | $(n\text{-}butyl)_3P$ | 80.0 | 91 |
| G | $(ethyl)_2phenylP$ | 70.0 | 82 |
| H | $(benzyl)_3P$ | 80.0 | 91 |

Example 15

The reaction temperature of the process can influence rate and selectivity. Rate increases with increasing temperature, but selectivity decreases with increasing temperature as is illustrated by the data below. At temperatures above about 60°C some 1,3,7-octatriene is produced.

To an 80 ml glass-lined autoclave were charged palladium acetate (6 mg), tri(iso-propyl)phosphine (16.5 mg), triethylamine-formic acid salt (2.5 g), pyridine (10 ml) and 1,3-butadiene (2 g). The stirred reactor was heated for the times and temperatures given below and the results were analyzed by gas chromatography.

Table III

| Temp. °C | Time, hrs | Butadiene Conv., % | Selectivity % to 1,7-Octadiene | 1,3,7-Octatriene |
|---|---|---|---|---|
| 100 | 0.5 | 90 | 92 | 3.1 |
| 70 | 1.0 | 96 | 96 | 0.8 |
| 60 | 2.0 | 94 | 97 | 0.2 |
| 40 | 4.0 | 98 | 98 | 0.0 |
| 50 | 16.0 | 16 | 98 | 0.0 |

Examples 16—32

The effects of various solvents on the rates of conversion of 1,3-butadiene was tested. To an 80 ml glass-lined autoclave were charged palladium nitrate ($2.7\times10^{-5}$ mol, as a 10% aqueous solution), tri(iso-propyl)-phosphine ($5.4\times10^{-5}$ mol), triethylamine-formic acid salt (2.5 g), 1,3-butadiene (2 g) and the appropriate solvent (1 ml).

The stirred reactor was heated for 1 hour at 40°C and the results were analyzed by gas chromatography and are given below.

| Example | Solvent | Butadiene Conversion, % |
|---|---|---|
| 16 | None | 9.7 |
| 17 | Pyridine | 20.5 |
| 18 | Tetrahydrofuran | 26.7 |
| 19 | 1,4-Butanediol | 0.8 |
| 20 | Ethylene glycol | 5.2 |
| 21 | 2,4-Pentanedione | 4.6 |
| 22 | Toluene | 35.5 |
| 23 | Acetic anhydride | 30.0 |
| 24 | Methyl formate | 32.6 |
| 25 | Ethylene carbonate | 99.0 |
| 26 | Propylene carbonate | 100.0 |
| 27 | Nitrobenzene | 65.0 |
| 28 | Nitromethane | 100.0 |
| 29 | Benzonitrile | 73.0 |
| 30 | Dimethylformamide | 83.0 |
| 31 | Sulfolane | 88.0 |
| 32 | Dimethylsulphoxide | 86.0 |

## Claims

1. A process for preparing 1,7-octadiene by hydrodimerizing 1,3-butadiene in the presence of a catalytic amount of palladium or a palladium compound, a tertiary phosphine, formic acid, a base which can neutralize formic acid and optionally a solvent, characterized in that the tertiary phosphine has the formula:

$$R_1R_2R_3P$$

wherein $R_1$ is a branched alkyl group having from 3 to 10 carbon atoms with branching occurring at a carbon atom no more than two carbon atoms from the phosphorus atom and $R_2$ and $R_3$ are $R_1$ or independently are alkyl, alkenyl or aryl groups having from 1 to 10 carbon atoms.

2. A process as claimed in claim 1, characterized in that the phosphine is tri-isopropylphosphine, tri-sec-butylphosphine, tri-isobutylphosphine, di(tert-butyl)-n-butylphosphine, di(tert-butyl)allylphosphine or tert-butyldi-n-propylphosphine.

3. A process as claimed in claim 1 or 2, characterized in that as solvent nitromethane, ethylene carbonate or propylene carbonate is used.

## Revendications

1. Procédé pour la préparation de, 1,7-octadiène par hydrodimérisation de 1,3-butadiène en présence d'une quantité catalytique de palladium ou d'un composé du palladium, d'une phosphine tertiaire, d'acide formique, d'une base qui peut neutraliser l'acide formique et éventuellement d'un solvent, caractérisé en ce que la phosphine tertiaire a la formule:

$$R_1R_2R_3P$$

dans laquelle $R_1$ est une groupe alcoyle ramifié ayant de 3 à 10 atomes de carbone, la ramification se trouvant à un atome de carbone qui n'est pas à plus de deux atomes de carbone de l'atome de phosphore et $R_2$ et $R_3$ sont tels que $R_1$ ou sont indépendamment des groupes alcoyle, alcényle ou aryle ayant de 1 à 10 atomes de carbone.

2. Procédé selon la revendication 1, caractérisé en ce que la phosphine est la tri-isopropylphosphine, la tri-sec-butylphosphine, la tri-isobutylphosphine, la di(tert-butyl)-n-butylphosphine, la di(tert-butyl)allylphosphine ou la tert-butyl-di-n-propylphosphine.

3. Procédé selon l'une des revendications 1 et 2, caractérisé en ce que comme solvant on utilise le nitrométhane, le carbonate d'éthylène ou le carbonate de propylène.

## Patentansprüche

1. Verfahren zur Herstellung von 1,7-Octadien durch Hydrodimerisierung von 1,3-Butadien in Gegenwart einer katalytischen Menge von Palladium oder einer Palladiumverbindung, eines tertiären Phosphins, von Ameisensäure, einer Base, die die Ameisensäure neutralisieren kann, und gegebenenfalls eines Lösungsmittels, dadurch gekennzeichnet, dass das tertiäre Phosphin die Formel:

$$R_1R_2R_3P$$

hat, in der $R_1$ ein verzweigter Alkylrest mit 3 bis 10 Kohlenstoffatomen ist, wobei sich die Verzweigung an einem Kohlenstoffatom befindet, das nicht weiter als 2 Kohlenstoffatome vom Phosphoratom entfernt ist, und $R_2$ und $R_3$ wie $R_1$ sind oder unabhängig voneinander Alkyl-, Alkenyl- oder Arylreste mit 1 bis 10 Kohlenstoffatomen sind.

2. Verfahren gemäss Anspruch 1, dadurch gekennzeichnet, dass das Phosphin Tri-isopropylphosphin, Tri-sek.-butylphosphin, Tri-isobutylphosphin, Di-(tert.-butyl)-n-butylphosphin, Di-(tert.-butyl)allylphosphin oder tert.-Butyl-di-n-propylphosphin ist.

3. Verfahren gemäss Anspruch 1 oder 2, dadurch gekennzeichnet, dass als Lösungsmittel Nitromethan, Äthylencarbonat oder Propylencarbonat verwendet wird.